(19) Europäisches Patentamt
European Patent Office
Office européen des brevets

(11) EP 0 924 191 A2

(12) EUROPÄISCHE PATENTANMELDUNG

(43) Veröffentlichungstag:
**23.06.1999 Patentblatt 1999/25**

(51) Int Cl.[6]: **C07C 209/64**

(21) Anmeldenummer: **98123147.5**

(22) Anmeldetag: **04.12.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **17.12.1997 DE 19756145**

(71) Anmelder: **Bayer Aktiengesellschaft
51368 Leverkusen (DE)**

(72) Erfinder:
- **Behre, Horst Dr.
51519 Odenthal (DE)**
- **Fiege, Helmut Dr.
51373 Leverkusen (DE)**
- **Eymann, Wolfgang Dr.
51061 Köln (DE)**
- **Arndt, Frank Dr.
47800 Krefeld (DE)**
- **Wiemers, Rudolf Dr.
40668 Meerbusch (DE)**
- **Klausener, Alexander Dr.
50259 Pulheim (DE)**

# (54) Herstellung von N-Phenyl-1-naphthylamin

(57) N-Phenyl-1-naphthylamin kann durch Umsetzung von Anilin und 1-Naphthylamin in fllüssiger Phase bei 100-400°C und unter Normaldruck hergestellt werden, wobei ein Bor und Fluor enthaltendes Katalysatorgemisch eingesetzt wird. Solche Katalysatorgemische können beispielsweise durch Umsetzung von Fluorwasserstoff, Borsäure und Anilin und/oder 1-Naphthylamin erhalten werden. Der Katalysator wird wiedergewonnen und kann erneut eingesetzt werden. Die Reaktion kann diskontinuierlich oder kontinuierlich durchgeführt werden.

EP 0 924 191 A2

**Beschreibung**

Die vorliegende Erfindung betrifft ein Verfahren zur Herstellung von N-Phenyl-1-naphthylamin (Phenyl-α-naphthylamin) der Formel (I)

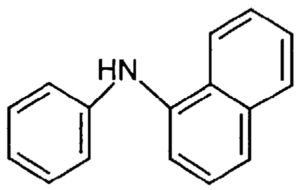

(I)

durch Umsetzung von Anilin mit 1-Naphthylamin, worin die Reaktion in Gegenwart eines Bor und Fluor enthaltenden Katalysators in kondensierter Phase und unter normalem Umgebungsdruck durchgeführt wird.

N-Phenyl-1-naphthylamin wird beispielsweise als Alterungs- oder Oxidationsschutzmittel für Kautschuk sowie für Mineralölprodukte eingesetzt (Ullmanns Encyklopädie der technischen Chemie, 4. Auflage, Bd. 17, S. 107, Verlag Chemie, Weinheim 1979). Weitere Einsatzmöglichkeiten finden sich auf dem Gebiet der Farbstoffe. So erhält man etwa den Farbstoff Victoriablau, ein Diphenylnaphthylmethan-Derivat, ausgehend von N-Phenyl-1-naphthylamin (H. E. Fierz-David und L. Blangley, Farbenchemie, 8. Aufl., S. 171, Springer-Verlag, Wien 1952). Auch Derivate des N-Phenyl-1-naphthylamins, die entweder aus dieser Verbindung selbst oder auf analoge Weise zu dieser Verbindung hergestellt werden, sind als Komponenten von Schmiermitteln von Interesse (EP 716 141).

In der Literatur sind verschiedentlich Verfahren zur Herstellung von N-Phenyl-1-naphthylamin beschrieben worden. So wird nach BIOS Final Report 986, II, S. 363, N-Phenyl-1-naphthylamin durch die Umsetzung von Anilin und 1-Naphthylamin bei Temperaturen von 230-250°C in Gegenwart katalytischer Mengen p-Toluolsulfonsäure erhalten. Dabei werden Ausbeuten von bis zu 91 % der theoretischen Ausbeute erzielt. Nachteilhaft ist bei diesem Verfahren, daß der Katalysator offenbar nicht wiederverwendet werden kann, was unter ökonomischen und ökologischen Gesichtspunkten als unbefriedigend gewertet werden muß. Nach H. E. Fierz-David und L. Blangley, Farbenchemie, 8. Aufl., S. 171, Springer-Verlag, Wien 1952, erhält man N-Phenyl-1-naphthylamin in Ausbeuten von 86-91 % der theoretischen Ausbeute durch Umsetzung von Anilin und 1-Naphthylamin bei Temperaturen von 195-215°C, wobei Sulfanilsäure als Katalysator dient. Wie in dem zuvor zitierten Verfahren erfolgt auch hier keine Wiedergewinnung des Katalysators, sodaß die ökonomischen und ökologischen Nachteile dieselben sind. Nach einer in DE-PS 241 853 sowie in J. Prakt. Chem. 89, 1 (1914) beschriebenen Vorgehensweise erhält man N-Phenyl-1-naphthylamin durch Umsetzung von Anilin und 1-Naphthylamin bei Temperaturen von 225-250°C in Gegenwart katalytischer Mengen Jod. Auch bei dieser Variante wird der Katalysator nicht wiederverwendet, so daß sich auch hier ökonomische und ökologische Nachteile ergeben. Die beschriebenen Reaktionsausbeuten sind zudem deutlich geringer als in den o.g. Verfahren. Nach J. Prakt. Chem. 89, 1 (1914) kann N-Phenyl-1-naphthylamin auch durch Umsetzung von 1-Naphthol mit Anilin bei Temperaturen zwischen 180 und 200°C ebenfalls in Gegenwart katalytischer Mengen Jod gewonnen werden. Die Ausbeuten erreichen jedoch nur 35-40 % der theoretischen Ausbeute, was diese Variante deutlich ungünstiger als die oben beschriebenen erscheinen läßt.

Allen genannten Verfahren ist gemeinsam, daß in ihrem Verlauf mehr oder weniger große Mengen unerwünschter Nebenprodukte, wie beispielsweise Diphenylamin, N-Phenyl-2-naphthylamin sowie 2-Naphthylamin, gebildet werden. Vor allem die letztgenannten Verbindungen N-Phenyl-2-naphthylamin und 2-Naphthylamin können grundsätzlich durch Isomerisierung von bereits gebildetem N-Phenyl-1-naphthylamin bzw. nicht umgesetztem 1-Naphthylamin entstehen, und zwar in Abhängigkeit von den zur Anwendung kommenden Reaktionsbedingungen und dem verwendeten Katalysator. Abgesehen davon, daß die Bildung derartiger unerwünschter Nebenprodukte aufgrund damit verbundener Materialverluste und dem erforderlich werdenden erhöhten trenntechnischen Aufwand zur Isolierung von reinem N-Phenyl-1-naphthylamin eine erhebliche Beeinträchtigung der Wirtschaftlichkeit des jeweiligen Herstellungsprozesses nach sich zieht, ist ferner zu berücksichtigen, daß insbesondere 2-Naphthylamin stark toxische Eigenschaften besitzt und seine Entstehung daher möglichst auszuschließen ist.

Es bestand somit die Aufgabe, ein wirtschaftliches Verfahren zur hochselektiven Herstellung von N-Phenyl-1-naphthylamin zu finden, bei dem die Bildung unerwünschter Nebenprodukte, vor allem aber die Bildung von 2-Naphthylamin, weitestgehend ausgeschlossen ist und das die Rückführung des zur Anwendung kommenden Katalysators erlaubt.

Es wurde überraschenderweise gefunden, daß sich N-Phenyl-1-naphthylamin in hohen Ausbeuten und ausgezeichneten Selektivitäten erhalten läßt, wenn man Anilin und 1-Naphthylamin in Gegenwart eines Fluorwasserstoff, Borsäure und Anilin und/oder 1-Naphthylamin enthaltenden Katalysators miteinander umsetzt. Weiterhin überraschend war die Beobachtung, daß der erfindungsgemäß zum Einsatz kommende Katalysator auf einfache Weise, also ohne Reinigung und Aufarbeitung, durch wäßrige Extraktion des ausreagierten Reaktionsgemisches wiedergewonnen werden kann. Der solchermaßen wiedergewonnene Katalysator kann in die Reaktion zurückgeführt werden, ohne daß dies zu unakzeptablen negativen Folgen für Umsatz und Selektivität führen würde. Angesichts des oben geschilderten Standes der Technik konnten diese günstigen Ergebnisse nicht erwartet werden. Vielmehr hätte man bei der Umsetzung von Anilin und 1-Naphthylamin mit der Bildung größerer Mengen unerwünschter Nebenprodukte und einem Verlust des Katalysators nach Durchführung der Reaktion rechnen müssen.

Es wurde ein Verfahren zur Herstellung von N-Phenyl-1-naphthylamin durch Umsetzung von Anilin und 1-Naphthylamin in flüssiger Phase bei 100-400°C und unter normalem Umgebungsdruck gefunden, das dadurch gekennzeichnet ist, daß man die Reaktion in Gegenwart eines Bor und Fluor enthaltenden Katalysatorgemisches durchführt.

Als Katalysatoren für das erfindungsgemäße Verfahren zur Herstellung von N-Phenyl-1-naphthylamin lassen sich entweder frisch hergestellte Katalysatorchargen oder wiedergewonnene Katalysatormengen, die im Zuge der Aufarbeitung von Reaktionsgemischen erhalten werden, verwenden. Grundsätzlich ist es auch möglich, Gemische aus wiedergewonnenem und frisch hergestelltem Katalysator zum Einsatz zu bringen. Als Katalysator für das erfindungsgemäße Verfahren wird im Falle des Einsatzes frischer Katalysatorchargen bevorzugt das Reaktionsprodukt verwendet, daß bei der Umsetzung von Fluorwasserstoff, Borsäure und Anilin und/oder 1-Naphthylamin erhalten wird. Hierbei handelt es sich um ein salzartiges Produkt der Formel

$$[A]\,[B] \qquad (II),$$

in der

A für die Kationen $[H]^+$, $[NH_4]^+$, $[Phenyl\text{-}NH_3]^+$, $[(1\text{-}Naphthyl)\text{-}NH_3]^+$, $[Phenyl\text{-}NH_2\text{-}Phenyl]^+$ oder für $[Phenyl\text{-}NH_2\text{-}(1\text{-}Naphthyl)]^+$, bevorzugt für die Kationen $[H]^+$, $[NH_4]^+$, $[Phenyl\text{-}NH_3]^+$ und $[(1\text{-}Naphthyl)\text{-}NH_3]^+$ und besonders bevorzugt für die Kationen $[NH_4]^+$ und $[Phenyl\text{-}NH_3]^+$ steht und

B für Anionen des Typs $[B(OH)_nF_{4-n}]^-$, in der n ganzzahlige Werte von 0 bis 4 annehmen kann, und bevorzugt für das Anion $[BF_4]^-$ steht.

Wichtige Einzelverbindungen der Formel (II) sind etwa $[NH_4]^+[BF_4]^-$ und $[Phenyl\text{-}NH_3]^+[BF_4]^-$.

In der Praxis können die innerhalb der erfindungsgemäßen Reaktion wirksamen Katalysatoren, im folgenden vereinfacht "erfindungsgemäßer Katalysator" genannt, Gemische verschiedener der allgemeinen Formel (II) entsprechenden Komponenten sein, deren Zusammensetzung sich jedoch nach Maßgabe der stöchiometrischen Verhältnisse sowie der Basizitäten und Dissoziationskonstanten der gegebenenfalls beteiligten kationischen Hauptkomponenten Wasser, Ammoniak, Anilin, 1-Naphthylamin und N-Phenyl-1-naphthylamin einstellen und die im allgemeinen Anteile der Komponenten Wasser, Ammoniak, Anilin und/oder 1-Naphthylamin sowie gegebenenfalls noch weitere aromatische Diamine auch in unprotonierter Form enthalten. Diese Gemische liegen unter den Reaktionsbedingungen zur Herstellung von N-Phenyl-1-naphthylamin in homogen gelöster Form vor, wirken demnach also in ihrer Gesamtheit als homogenes Katalysatorsystem.

Die Herstellung von frischen Chargen des erfindungsgemäßen Katalysators erfolgt im allgemeinen unter Anwendung von dem Fachmann bekannten Verfahren. So ist es beispielsweise möglich, wäßrige Lösungen von Ammoniumtetrafluoroborat als Frischkatalysator einzusetzen. In einer bevorzugten Ausführungsform des erfindungsgemäßen Verfahrens wird die Herstellung frischer Chargen des erfindungsgemäßen Katalysators in räumlich getrennten, gesonderten Reaktionsgefäßen durchgeführt. Beispielsweise kann die Herstellung des erfindungsgemäßen Katalysators dadurch erfolgen, daß man als Reaktionskomponenten Anilin und/oder 1-Naphthylamin, Borsäure und Fluorwasserstoff miteinander zur Reaktion bringt. Bevorzugt werden Anilin, Borsäure und Fluorwasserstoff miteinander zur Reaktion gebracht. Für die Herstellung des erfindungsgemäßen Katalysators wird bevorzugt frisch zugeführtes Anilin und/oder 1-Naphthylamin eingesetzt. Es kann aber auch von Vorteil sein, hierfür bei der Aufarbeitung von ausreagierten Reaktionsgemsichen anfallendes zurückgewonnenes Anilin und/oder 1-Naphthylamin einzusetzen.

Die für die Herstellung des erfindungsgemäßen Katalysators verwendete Borsäure wird bevorzugt als Feststoff eingesetzt. Grundsätzlich ist es aber auch möglich und kann gegebenenfalls von Vorteil sein, die Borsäure als Schmelze, als Lösung oder als Aufschlämmung, beispielsweise in Wasser und/oder Anilin, zum Einsatz zu bringen.

Der für die Herstellung des erfindungsgemäßen Katalysators verwendete Fluorwasserstoff wird vorzugsweise als wäßrige Lösung zum Einsatz gebracht. In einer bevorzugten Ausführungsform setzt man den benötigten Fluorwasserstoff als konzentrierte wäßrige Lösung ein. Grundsätzlich sind aber auch andere Dosierweisen möglich, so beispielsweise die Einleitung von gasförmigem oder verflüssigtem reinen Fluorwasserstoff.

Zur Herstellung des erfindungsgemäßen Katalysators werden die beteiligten Komponenten im Gewichtsverhältnis Anilin und/oder 1-Naphthylamin:Borsäure:Fluorwasserstoff= (100 bis 2000) : (50 bis 250) : (50-250) zum Einsatz gebracht. Bevorzugt bringt man Anilin, Borsäure und Fluorwasserstoff im Gewichtsverhältnis von (300 bis 1000) : (100 bis 150) : (100 bis 150) zum Einsatz. Die Reihenfolge der Dosierung der genannten Komponenten ist grundsätzlich frei wählbar. In einer bevorzugten Ausführungsform wird zunächst Anilin vorgelegt, danach Borsäure hinzugefügt und schließlich wäßrige Fluorwasserstoffsäure eindosiert.

Die Herstellung des erfindungsgemäßen Katalysators erfolgt im allgemeinen bei einer Reaktionstemperatur zwischen 0 und 250°C, bevorzugt zwischen 30 und 200°C, besonders bevorzugt zwischen 70 und 150°C.

Bei der Herstellung des erfindungsgemäßen Katalysators kann es zweckmäßig sein zu rühren. Grundsätzlich ist es aber auch möglich, auf zusätzliches Rühren zu verzichten. Die im Verlaufe der Herstellung des erfindungsgemäßen Katalysators freiwerdende Reaktionswärme kann ganz oder teilweise durch äußere Kühlung abgeführt werden. Im allgemeinen erfolgt die Herstellung des erfindungsgemäßen Katalysators bei Normaldruck. Grundsätzlich ist es aber auch möglich, die Umsetzung bei erniedrigtem oder erhöhtem Druck durchzuführen.

Der solchermaßen hergestellte erfindungsgemäße Katalysator wird im allgemeinen in flüssiger Form ohne Isolierung oder weitere Aufreinigung in die Reaktion zur Herstellung von N-Phenyl-l-naphthylamin eingesetzt. Grundsätzlich ist es aber auch möglich, den erfindungsgemäßen Katalysator etwa unter reduziertem Druck zur Trockne einzudampfen und den dabei anfallenden festen Rückstand als katalytisch wirksames Gemisch in die Reaktion zur Herstellung von N-Phenyl-1-naphthylamin einzusetzen.

Das erfindungsgemäße Verfahren beinhaltet zunächst die in der Flüssigphase innerhalb eines geeigneten Reaktors in Gegenwart des erfindungsgemäßen Katalysators ablaufende Umsetzung von Anilin mit 1-Naphthylamin. Das nach Reaktionsende anfallende flüssige Produktgemisch wird mit Wasser und/oder einer wäßrigen Lösung des erfindungsgemäßen Katalysators versetzt und in einer hierzu geeigneten Apparatur in eine überwiegend wäßrige und eine überwiegend organische flüssige Phase aufgetrennt.

Die den größten Teil des organischen Materials enthaltende flüssige Phase, die im wesentlichen das Reaktionsprodukt N-Phenyl-1-naphthylamin, nicht umgesetztes Ausgangsmaterial, also Anilin und 1-Naphthylamin, sowie kleine Anteile an Nebenprodukten enthält, wird abgetrennt und der weiteren Aufarbeitung zugeführt. Die den größten Teil des salzartigen Katalysators enthaltende überwiegend wäßrige Phase wird ebenfalls abgetrennt. Sie kann ganz oder teilweise ausgeschleust werden und/oder ganz oder teileweise ohne weitere Reinigung oder Aufarbeitung erneut als Katalysator für die Durchführung der erfindungsgemäßen Umsetzung von Anilin und 1-Naphthylamin Verwendung finden.

Die den größten Teil des organischen Materials enthaltende flüssige Phase wird gegebenenfalls noch ein oder mehrere weitere Male unter Zusatz von Wasser sowie gegebenenfalls eines oder mehrerer zusätzlicher anorganischer Hilfsstoffe gewaschen und danach der destillativen Aufarbeitung unterzogen. Bei der abschließenden destillativen Aufarbeitung des Reaktionsproduktes fällt N-Phenyl-1-naphthylamin in sehr reiner Form an. Überschüssiges Anilin und/oder 1-Naphthylamin wird gleichfalls in einer solchen Güte erhalten, daß sich beide Komponenten problemlos in den Herstellungsprozeß zurückführen lassen. Geringe Mengen Destillationsrückstände werden einer Reststoffverwertung zugeführt oder verworfen.

Die destillative Aufarbeitung kann auf an sich bekannte Weise erfolgen. Dabei ist grundsätzlich eine kontinuierliche oder eine diskontinuierliche Vorgehensweise möglich. Die einzelnen unerwünschten Komponenten, wie beispielsweis verbliebenes Wasser, überschüssige Reaktanden oder Nebenprodukte, können etwa durch fraktionierte Destillation aus einer Destillationsblase heraus oder mittels mehrerer hintereinander geschalteter Destillationsapparaturen abgetrennt werden. Dabei können grundsätzlich alle dem Fachmann bekannten und für die genannte Trennaufgabe geeigneten Destillationsapparaturen zum Einsatz kommen.

Die Durchführung des erfindungsgemäßen Verfahrens zur Herstellung von N-Phenyl-1-naphthylamin kann grundsätzlich in ihrer Gesamtheit oder in Teilbereichen auf diskontinuierliche oder auf kontinuierliche Weise erfolgen. Bevorzugt erfolgt die eigentliche Umsetzung von Anilin mit 1-Naphthylamin auf diskontinuierliche Weise; die nachfolgende Aufarbeitung kann sich aber in kontinuierlicher Weise anschließen.

Die außerordentlich hohe Effektivität des erfindungsgemäßen Verfahrens ist überraschend und wird in der Literatur nicht vorbeschrieben. Im Gegenteil wurden kompliziertere bzw. gegenüber Nebenreaktionen bedeutend störanfälligere Operationen wie beispielsweise die oben diskutierten Alternativverfahren vorgeschlagen.

Die zur Herstellung von N-Phenyl-1-naphthylamin eingebrachten Reaktanden Anilin und 1-Naphthylamin werden in einem molaren Verhältnis eingesetzt, das bei 1 : 3 bis 10 : 1, bevorzugt bei 1 : 2 bis 5 : 1 und besonders bevorzugt bei 1 : 1 bis 3 : 1 liegt. Dabei ist es belanglos, in welchem Aggregatzustand die jeweiligen Komponenten in die Reaktion eingebracht werden. In einer bevorzugten Ausführungsform werden sowohl Anilin als auch 1-Naphthylamin in flüssiger Form in die Reaktion eingebracht. Es kann aber auch von Vorteil sein, etwa 1-Naphthylamin als Feststoff oder Anilin in verdampfter Form in das Reaktionsgemisch einzubringen.

Der entsprechend dem erfindungsgemäßen Verfahren in die Reaktion zur Herstellung von N-Phenyl-1-naphthylamin eingebrachte Katalysator bzw. das Katalysatorgemisch wird in einer solchen Menge eingesetzt, daß das molare Verhältnis zwischen der im Katalysator bzw. dem Katalysatorgemisch vorhandenen Stoffmenge Bor und der gesamten Stoffmenge des in die Reaktion eingesetzten Anilins und 1-Naphthylamins zwischen 1 : 10 und 1 : 100.000, bevorzugt zwischen 1 : 25 und 1 : 10.000 und besonders bevorzugt zwischen 1 : 50 und 1 : 1.000 liegt. Der erfindungsgemäße Katalysator bzw. das Katalysatorgemisch wird im allgemeinen in flüssiger Form in das Reaktionsgemisch eingebracht. Grundsätzlich ist es aber auch möglich, den erfindungsgemäßen Katalysator bzw. das Katalysatorgemisch nach Eindampfen katalysatorhaltiger Lösungen, wie sie etwa bei der Herstellung frischer Katalysatorchargen oder bei der Wiedergewinnung von Katalysator im Zuge der weiteren Aufarbeitung ausreagierter Reaktionsgemische anfallen, in fester Form als katalytisch wirksames Gemisch in die erfindungsgemäße Reaktion zur Herstellung von N-Phenyl-1-naphthylamin einzubringen.

Die erfindungsgemäße Reaktion zur Herstellung von N-Phenyl-1-naphthylamin wird bei normalem Umgebungsdruck durchgeführt. Die erfindungsgemäße Reaktion zur Herstellung von N-Phenyl-1-naphthylamin wird im Temperaturbereich zwischen 100 und 400°C, bevorzugt zwischen 150 und 300°C, besonders bevorzugt zwischen 180 und 250°C, durchgeführt. Zur Gewährleistung des gewählten Temperaturniveaus wird dem Reaktionsgefäß durch außen- und/oder innenliegende Heizaggregate die erforderliche Wärmeenergie zugeführt. In einer bevorzugten Ausführungsform des erfindungsgemäßen Verfahrens arbeitet man bei der sich unter den gegebenen Verhältnissen frei einstellenden Siedetemperatur des Reaktionsgemisches, die mit fortschreitendem Umsatz durch Abnahme des Leichtsiederanteils und Zunahme des Schwersiederanteils tendenziell ansteigt.

Das Reaktionsgut wird im Verlaufe der Reaktion zur Herstellung von N-Phenyl-1-naphthylamin durchmischt. Dies kann beispielsweise durch Rühraggregate, durch außen- oder innenliegende Umpumpvorrichtungen oder auf eine andere geeignete Weise geschehen. Grundsätzlich ist es aber auch möglich, im Verlaufe der Reaktion aufjegliche aktive Durchmischung des Reaktionsgutes zu verzichten, da das im Zuge der Umsetzung der Reaktanden freiwerdende Ammoniakgas sowie das Sieden des Reaktionsgemisches ebenfalls zu einer Durchmischung führen.

Das im Zuge der Umsetzung der Reaktanden entweichende Ammoniakgas wird abgetrennt und einer weiteren Verwertung oder einer geregelten Entsorgung zugeführt. Die Abtrennung des im Zuge der Umsetzung der Reaktanden entweichenden Ammoniakgases erfolgt im allgemeinen mit Hilfe dazu dem Fachmann bekannter geeigneter Kondensatoren, deren Betriebsbedingungen so gewählt werden, daß sie den Durchtritt des Ammoniakgases erlauben, die erheblich höher siedenden Reaktanden sowie die Reaktionsprodukte jedoch im Reaktionsbereich zurückhalten.

Neben den genannten Reaktionsbedingungen sowie den apparativen Parametern ist die zeitliche Dauer der erfindungsgemäßen Reaktion zur Herstellung von N-Phenyl-1-naphthylamin maßgeblich für den erzielten Umsetzungsgrad der eingesetzten Reaktanden Anilin und 1-Naphthylamin. Da das erfindungsgemäße Verfahren zur Herstellung von N-Phenyl-1-naphthylamin die Rückführung nicht umgesetzten Anilins und/oder 1-Naphthylamins ermöglicht, ist die Reaktionszeit in weiten Grenzen variierbar und kann unter prozeßökonomischen Gesichtspunkten beispielsweise so gewählt werden, daß die Raum-Zeit-Ausbeuten des Reaktionsteils des erfindungsgemäßen Verfahrens möglichst hoch und der trenntechnische Aufwand zur Rückführung nicht umgesetzten Ausgangsmaterials möglichst klein ist. Im allgemeinen werden mehr als 50 %, bevorzugt mehr als 60 %, besonders bevorzugt mehr als 70 % des bei diskontinuierlicher Verfahrensweise in einen diskreten Reaktionsansatz bzw. bei kontinuierlicher Verfahrensweise in die Reaktionszone eingebrachten 1-Naphthylamins umgesetzt.

Das nach Beendigung der Reaktion anfallende Gemisch wird, bevorzugt nach Abkühlen auf eine Temperatur unterhalb von 100°C, zunächst mit Wasser und/oder wasserhaltiger Lösung wiedergewonnenen Katalysatorgemisches aus vorangegangenen Aufarbeitungen versetzt und vermischt. Das dabei anfallende zweiphasige Gemisch wird einer Phasentrennung zugeführt. Die Menge des in diesen Extraktionsprozeß eingesetzten Wassers und/oder der wasserhaltigen Lösung aus vorangegangenen Aufarbeitungen wiedergewonnenen Katalysatorgemisches wird vorzugsweise so gewählt, daß der nach der genannten Phasentrennung schließlich anfallende wäßrige Extrakt ca. 10 - 35 % nicht verdampfbare katalytisch wirksame Komponenten enthält. Die Temperatur des zweiphasigen Gemisches wird im Verlauf der Extraktion und der Phasentrennung im allgemeinen im Bereich zwischen 40 und 100°C gehalten.

Die nach der genannten Phasentrennung erhaltene organische Phase wird vorzugsweise einer weiteren Extraktion unterworfen. Grundsätzlich ist es aber auch möglich, sie ohne weitere Behandlung aufzudestillieren. Die zweite Extraktion erfolgt mit Wasser, dem gegebenenfalls zwischen 0 und 50 %, bevorzugt zwischen 5 und 30 %, besonders bevorzugt zwischen 10 und 20 % eines oder mehrerer Hilfsstoffe, bevorzugt eines oder mehrerer salzartiger anorganischer Hilfsstoffe, besonders bevorzugt eines oder mehrerer Stoffe aus der Gruppe der Alkali- und Erdalkalimetallhalogenide, der Alkalimetallsulfate, der Alkalimetallcarbonate und der Alkalimetallhydrogencarbonate zugesetzt wurde. Die Temperatur des zweiphasigen Gemisches wird im Verlauf der Extraktion und der Phasentrennung im allgemeinen im Bereich zwischen 30 und 90°C gehalten. Das dabei anfallende zweiphasige Gemisch wird einer Phasentrennung zugeführt. Die nach der genannten Phasentrennung erhaltene wäßrige Phase wird zum Teil für weitere Extraktionen wiederverwendet, zum Teil ausgeschleust und einer Verwertung oder einer geregelten Entsorgung zugeführt. Die nach der Phasentrennung erhaltene organische Phase wird der destillativen Auftrennung zugeführt. Beispielsweise mit Hilfe konventioneller, dem Fachmann bekannter Destillationsapparaturen und Destillationstechniken wird diese destillative Auftrennung vorzugsweise so durchgeführt, daß in dem rohen Destillationsgemisch vorhandenes nicht umgesetztes Anilin und 1-Naphthylamin vollständig wiedergewonnen und in spätere Reaktionen zur Herstellung von N-Phenyl-1-naphthylamin wiedereingesetzt werden können.

Beispiele

**Beispiel 1**

Zu einem Gemisch aus 82,00 kg (880,49 mol) Anilin und 72,00 kg (502,83 mol) 1-Naphthylamin gab man 3,00 kg (6,01 mol) einer 21,0 %igen Lösung von Ammoniumtetrafluoroborat in Wasser. Man erhitzte ca. 2 Stunden lang unter einem Druck von 20 mbar auf eine Temperatur von ca. 100°C, wobei ca. 10 kg eines Gemisches aus Anilin und Wasser abdestillierten. Man fügte weitere 10,00 kg (107,38 mol) Anilin zu dem Reaktionsgemisch hinzu und erhitzte nun während 24 Stunden unter Normaldruck auf Temperaturen zwischen 200 und 215°C. Siedendes Anilin wurde durch einen aufgesetzten Kondensator am Entweichen gehindert, während das entstehende Ammoniakgas am Kopf des Kondensators abgeleitet wurde. Nach beendeter Umsetzung ließ man das Reaktionsgemisch auf ca. 100°C abkühlen und versetzte es mit 17,00 kg einer 18 %igen Lösung von Ammonium-tetrafluoroborat in Wasser. Nach intensiver Durchmischung bei Temperaturen zwischen 60 und 80°C ließ man absitzen. Die untere Phase wurde abgetrennt und bei Temperaturen oberhalb von 50°C gelagert. Sie ließ sich als katalytisch wirksames Gemisch bei nachfolgenden Reaktionsansätzen wiederverwenden. Die obere Phase wurde mit 17,00 kg einer ca. 15 %igen Lösung von Natriumcarbonat in Wasser versetzt. Nach intensiver Durchmischung bei Temperaturen zwischen 60 und 80°C ließ man absitzen. Die dabei anfallende untere Phase wurde abgetrennt und verworfen. Die anfallende obere Phase wurde nach ihrer Abtrennung einer fraktionierten Destillation bei ca. 2 mbar unterworfen. Man erhielt nach vollständiger destillativer Trennung:

1. 47,75 kg nicht umgesetztes Anilin, das noch Wasserreste enthielt;

2. 7,35 kg nicht umgesetztes 1-Naphthylamin, das noch geringe Mengen Nebenprodukte, wie beispielsweise Diphenylamin, enthielt;

3. 2,89 kg rohes N-Phenyl-1-naphthylamin;

4. 93,37 kg reines N-Phenyl-1-naphthylamin, Analytik: GC 99,88 %, Gehalt an 2-Naphthylamin < 1 ppm;

5. 1,84 kg Destillationsrückstände:

Die Fraktionen 1., 2, und 3, ließen sich als Ausgangsmaterial für weitere Reaktionsansätze verwenden. Die Ausbeute an reinem N-Phenyl-1-naphthylamin betrug, bezogen auf umgesetztes 1-Naphthylamin, 94,1 % der theoretischen Ausbeute. Unter Einschluß der Rohfraktion 3. betrug die Ausbeute, bezogen auf umgesetztes l-Naphthylamin, 97,0 % der theoretischen Ausbeute.

**Beispiel 2**

Durchführung wie in Beispiel 1, wobei jedoch statt der wäßrigen Lösung von Ammoniumtetrafluoroborat ca. 3,5 kg der wäßrigen Lösung verwendet wurde, die in Beispiel 1 als erste untere Phase vom Reaktionsgemisch abgetrennt worden war. Die Ausbeute an reinem N-Phenyl-1-naphthylamin betrug 92,9 kg (bezogen auf umgesetztes 1-Naphthylamin = 93,6 % der theoretischen Ausbeute). Unter Einschluß der rohes N-Phenyl-1-naphthylamin enthaltenden Fraktionen betrug die chemische Ausbeute, bezogen auf umgesetztes 1-Naphthylamin, 96,5 % der theoretischen Ausbeute.

Analytik: GC 99,85 %, Gehalt an 2-Naphthylamin < 1 ppm

**Beispiel 3** (Herstellung von Katalysatorgemisch)

In einem Edelstahlkessel wurden 50,0 kg (536,9 mol) Anilin vorgelegt und bei Raumtemperatur während ca. 15 Minuten unter Rühren mit 12,0 kg (194,1 mol) Borsäure versetzt. Im Verlauf von ca. 120 Minuten ließ man unter Rühren 20,0 kg (729,6 mol) einer 73 %igen wäßrigen Flußsäurelösung zulaufen, während die Temperatur des Reaktionsgemisches vermittels äußerer Kühlung innerhalb eines Bereiches zwischen 20 und 60°C gehalten wurde.

**Beispiel 4**

Durchführung wie in Beispiel 1, wobei jedoch anstelle der wäßrigen Lösung von Ammoniumtetrafluoroborat insgesamt 3,0 kg des Katalysatorgemisches aus Beispiel 3 verwendet wurden. Die Ausbeute an reinem N-Phenyl-1-naphthylamin betrug 93,1 kg (bezogen auf umgesetztes 1-Naphthylamin = 93,8 % der theoretischen Ausbeute). Unter Einschluß der rohes N-Phenyl-1-naphthylamin enthaltenden Fraktionen betrug die chemische Ausbeute, bezogen auf umgesetztes 1-Naphthylamin, 96,7 % der theoretischen Ausbeute.

Analytik: GC 99,87 %, Gehalt an 2-Naphthylamin < 1 ppm

**Patentansprüche**

1. Verfahren zur Herstellung von N-Phenyl-1-naphthylamin durch Umsetzung von Anilin und 1-Naphthylamin in flüssiger Phase bei 100-400°C und unter normalem Umgebungsdruck, dadurch gekennzeichnet, daß man die Reaktion in Gegenwart eines Bor und Fluor enthaltenden Katalysatorgemisches

durchführt.

**2.** Verfahren nach Anspruch 1, dadurch gekennzeichnet, daß man die Reaktion zwischen 150 und 300°C, bevorzugt zwischen 180 und 250°C, durchführt.

**3.** Verfahren nach Ansprüchen 1 und 2, dadurch gekennzeichnet, daß man die Reaktion in Gegenwart eines durch Umsetzung von Fluorwasserstoff, Borsäure und Anilin und/oder 1-Naphthylamin erhaltenen Katalysatorgemisches durchführt.

**4.** Verfahren nach Anspruch 3, dadurch gekennzeichnet, daß die katalytisch wirksamen Komponenten des Katalysatorgemisches salzartige Verbindungen der Formel

$$[A]\ [B] \qquad (II)$$

sind, in der

A für die Kationen $[H]^+$, $[NH_4]^+$, $[Phenyl\text{-}NH_3]^+$, $[(1\text{-}Naphthyl)\text{-}NH_3]^+$, $[Phenyl\text{-}NH_2\text{-}Phenyl]^+$ oder für $[Phenyl\text{-}NH_2\text{-}(1\text{-}Naphthyl)]^+$, bevorzugt für die Kationen $[H]^+$, $[NH_4]^+$, $[Phenyl\text{-}NH_3]^+$ und $[(1\text{-}Naphthyl)\text{-}NH_3]^+$ und besonders bevorzugt für die Kationen $[NH_4]^+$ und $[Phenyl\text{-}NH_3]^+$ steht und

B für Anionen des Typs $[B(OH)_nF_{4-n}]^-$, in der n ganzzahlige Werte von 0 bis 4 annehmen kann, und bevorzugt für das Anion $[BF_4]^-$ steht.

**5.** Verfahren nach Anspruch 4, dadurch gekennzeichnet, daß die katalytisch wirksamen Komponenten des Katalysatorgemisches die Verbindungen $[NH_4]^+[BF_4]$ und $[Phenyl\text{-}NH_3]^+[BF_4]^-$ sind.

**6.** Verfahren nach Ansprüchen 1 bis 5, dadurch gekennzeichnet, daß man nach Beendigung der Umsetzung der Reaktanden die katalytisch wirksamen Komponenten durch Extraktion des erhaltenen Produktgemisches wiedergewinnt.

**7.** Verfahren nach Anspruch 6, dadurch gekennzeichnet, daß die Extraktion des erhaltenen Produktgemisches mit Wasser oder wasserhaltigen Lösungen der katalytisch wirksamen Komponenten durchgeführt wird.

**8.** Verfahren nach Anspruch 1, dadurch gekennzeichnet, daß die Durchführung der Reaktion auf diskontinuierliche Weise erfolgt.

**9.** Verfahren nach Anspruch 8, dadurch gekennzeichnet, daß die Durchführung der Reaktion auf diskontinuierliche Weise erfolgt, die Aufarbeitung aber kontinuierlich erfolgt.